# EUROPEAN PATENT SPECIFICATION

(11) **EP 4 216 894 B1**
(45) Date of publication and mention of the grant of the patent: **29.10.2025**
(21) Application number: 21798246.1
(22) Date of filing: 22.09.2021
(51) Int. Cl.: A61F 13/49, A61F 13/56, A61F 13/58

(54) **ABSORBENT ARTICLES WITH PATTERNED FRONT EARS**
SAUGFÄHIGE ARTIKEL MIT STRUKTURIERTEN VORDEREN OHREN
ARTICLES ABSORBANTS AYANT DES OREILLES AVANT À MOTIFS

(30) Priority: 22.09.2020 US 202063081484 P
(43) Date of publication of application: 02.08.2023
(73) Proprietor: The Procter & Gamble Company, Cincinnati, OH 45202 (US)
(72) Inventor: TALLY, Amy L., Cincinnati, Ohio 45202 (US); ARORA, Kelyn A., Cincinnati, Ohio 45202 (US)
(74) Representative: P&G Patent Germany
(86) International application number: PCT/US2021/071541
(87) International publication number: WO 2022/067310

(56) References cited:
- WO-A1-2019/191051
- WO-A2-2021/242593
- US-A1- 2007 093 768
- US-A1- 2015 173 958
- US-A1- 2020 100 949

## Description

### FIELD

The present disclosure is generally directed to absorbent articles with patterned front ears.

### BACKGROUND

Absorbent articles are used in the hygiene industry to contain and absorb bodily exudates (i.e., urine and bowel movements) in infants, toddlers, children, and adults. Absorbent articles may include, but not be limited to, taped diapers, pants, adult incontinence products, and feminine care products. Taped diapers (in all sizes for any wearers) may include front ears as e.g. disclosed in US2007093768, US2020100949, WO202124593, WO2019191051 and US2015173958. The front ears are used to provide better hip coverage and aid in application of the taped diapers. These front ears are typically flat, non-patterned, regular and relatively cheap nonwoven materials that are not aesthetically pleasing, and are sometimes hard to grasp. As such, front ears of taped diapers should be improved.

### SUMMARY

The present absorbent article comprises a front waist region, a back waist region, a liquid permeable topsheet, a liquid impermeable backsheet, and an absorbent core positioned at least partially intermediate the topsheet and the backsheet. The absorbent core comprises an absorbent material. The absorbent article comprises a pair of back ears in the back waist region and a pair of front ears in the front waist region. The present invention provides absorbent articles comprising patterned front ears that are soft and easy to grasp as indicated in the claims.

### BRIEF DESCRIPTION OF THE DRAWINGS

The above-mentioned and other features and advantages of the present disclosure, and the manner of attaining them, will become more apparent and the disclosure itself will be better understood by reference to the following description of example forms of the disclosure taken in conjunction with the accompanying drawings, wherein:
Fig. 1 is a plan view of an example absorbent article in the form of a taped diaper, garment-facing surface facing the viewer, in a flat laid-out state;
Fig. 2 is a plan view of the example absorbent article of Fig. 1, wearer-facing surface facing the viewer, in a flat laid-out state;
Fig. 3 is a front perspective view of the absorbent article of Figs. 1 and 2 in a fastened position;
Fig. 4 is a front perspective view of an absorbent article in the form of a pant;
Fig. 5 is a rear perspective view of the absorbent article of Fig. 4;
Fig. 6 is a plan view of the absorbent article of Fig. 4, laid flat, with a garment-facing surface facing the viewer;
Fig. 7 is a cross-sectional view of the absorbent article taken about line 7-7 of Fig. 6;
Fig. 8 is a cross-sectional view of the absorbent article taken about line 8-8 of Fig. 6;
Fig. 9 is a plan view of an example absorbent core or an absorbent article;
Fig. 10 is a cross-sectional view, taken about line 10-10, of the absorbent core of Fig. 9;
Fig. 11 is a cross-sectional view, taken about line 11-11, of the absorbent core of Fig. 10;
Fig. 12 is a plan view of an example absorbent article of the present disclosure that is a sanitary napkin;
Fig. 13 is an example cross-sectional view taken within a front waist region of an absorbent article;
Fig. 14 is an example cross-sectional view taken within a crotch region of an absorbent article;
Fig. 15 is an example cross-sectional view taken within a back waist region of an absorbent article;
Fig. 16 is a perspective view of a portion of an absorbent article comprising a front ear with a pattern comprising three-dimensional features;
Fig. 17 is a perspective view of a portion of an absorbent article comprising a front ear with a pattern comprising three-dimensional features;
Fig. 18 is a plan view of a front ear with a pattern comprising three-dimensional features;
Fig. 19 is a plan view of a portion of an absorbent article comprising a front ear with a pattern comprising three-dimensional features;
Fig. 20 is a perspective view of a portion of an absorbent article comprising a front ear with a pattern comprising visually distinguishable zones;
Fig. 21 is a plan view of a portion of an absorbent article comprising a front ear with a pattern comprising three-dimensional features;
Fig. 22 is a plan view of a front ear with a pattern comprising visually distinguishable zones;
Fig. 23 is a plan view of front ears with patterns comprising visually distinguishable zones;
Fig. 24 is a plan view of a front ear with a pattern comprising visually distinguishable zones;
Fig. 25 is a plan view of a portion of an absorbent article comprising front ears with patterns comprising three-dimensional features;
Fig. 26 is a plan view of a portion of an absorbent article comprising a front ear with a pattern comprising three-dimensional features covering only a portion of the front ear;
Fig. 27 is a plan view of a portion of an absorbent article comprising a front ear with a pattern comprising three-dimensional features covering only a portion of the front ear;
Fig. 28 is a plan view of a portion of an absorbent article comprising front ears with patterns comprising three-dimensional features and with the front ears having a non-white color or tint;
Fig. 29 is a plan view of a portion of an absorbent article comprising front ears with patterns comprising three-dimensional features and with the front ears having a non-white color or tint, wherein only a portion of the front ears have the three-dimensional features;
Fig. 30 is a plan view of a portion of an absorbent article comprising a front ear with a pattern comprising visually distinguishable zones with apertured areas and non-apertured areas;
Fig. 31 is a plan view of a portion of an absorbent article comprising a front ear with a pattern comprising visually distinguishable zones with apertured areas and non-apertured areas; and
Fig. 32 is a plan view of a portion of an absorbent article comprising a front ear with a pattern comprising visually distinguishable zones with apertured areas and non-apertured areas.

### DETAILED DESCRIPTION

Various non-limiting forms of the present disclosure will now be described to provide an overall understanding of the principles of the structure, function, manufacture, and use of the absorbent articles with patterned front ears disclosed herein. One or more examples of these non-limiting forms are illustrated in the accompanying drawings. Those of ordinary skill in the art will understand that the absorbent articles with patterned front ears described herein and illustrated in the accompanying drawings are non-limiting example forms and that the scope of the various non-limiting forms of the present disclosure are defined solely by the claims. The features illustrated or described in connection with one non-limiting form may be combined with the features of other non-limiting forms. Such modifications and variations are intended to be included within the scope of the present disclosure.

### General Description of an Absorbent Article

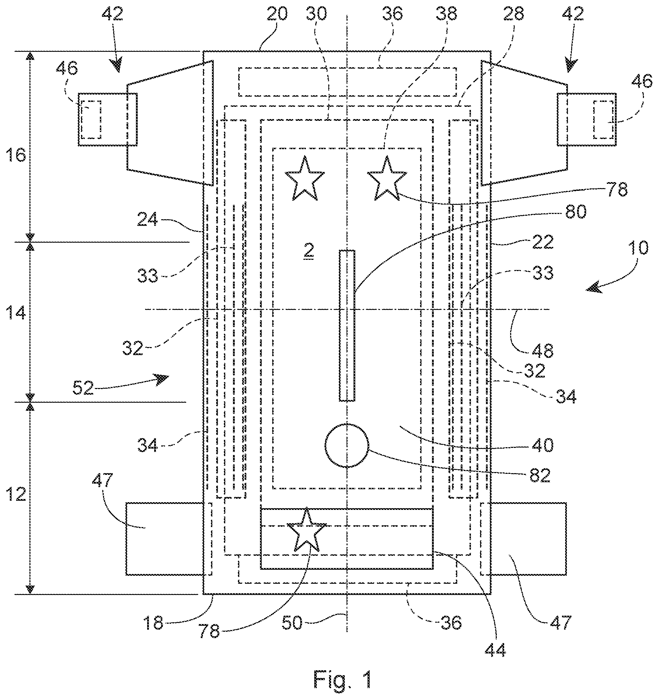

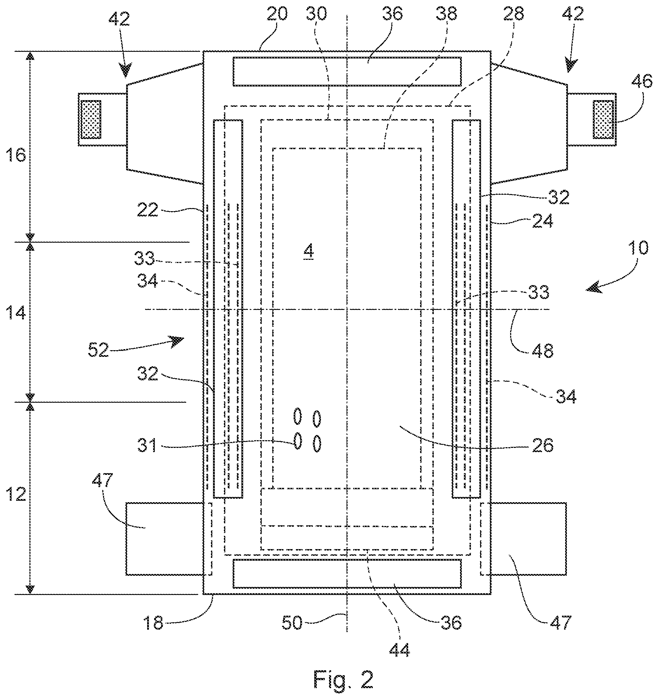

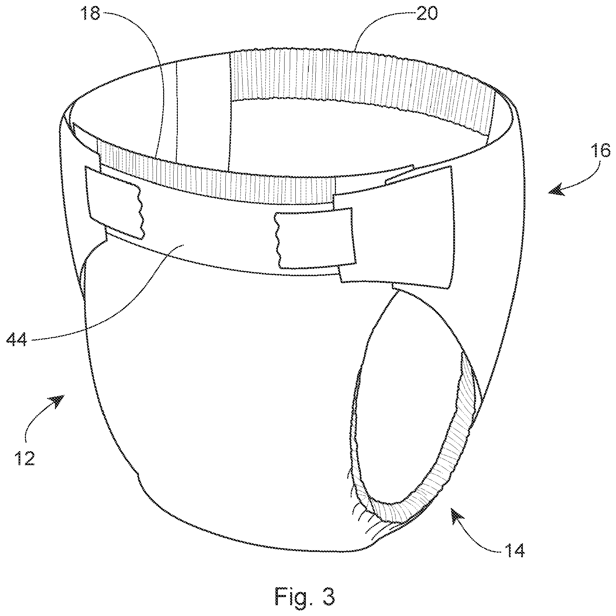

An example absorbent article 10 according to the present disclosure, shown in the form of a taped diaper, is represented in Figs. 1-3. Fig. 1 is a plan view of the example absorbent article 10, garment-facing surface 2 facing the viewer in a flat, laid-out state (i.e., no elastic contraction). Fig. 2 is a plan view of the example absorbent article 10 of Fig. 1, wearer-facing surface 4 facing the viewer in a flat, laid-out state. Fig. 3 is a front perspective view of the absorbent article 10 of Figs. 1 and 2 in a fastened configuration. The absorbent article 10 of Figs. 1-3 is shown for illustration purposes only as the present disclosure may be used for making a wide variety of diapers, including adult incontinence products, pants, or other absorbent articles, such as sanitary napkins and absorbent pads, for example.

The absorbent article 10 comprises a front waist region 12, a crotch region 14, and a back waist region 16. The crotch region 14 may extend intermediate the front waist region 12 and the back waist region 16. The front wait region 12, the crotch region 14, and the back waist region 16 may each be 1/3 of the length of the absorbent article 10. The absorbent article 10 may comprise a front end edge 18, a back end edge 20 opposite to the front end edge 18, and longitudinally extending, transversely opposed side edges 22 and 24 defined by the chassis 52.

The absorbent article 10 comprises a liquid permeable topsheet 26, a liquid impermeable backsheet 28, and an absorbent core 30 positioned at least partially intermediate the topsheet 26 and the backsheet 28. The absorbent article 10 may also comprise one or more pairs of barrier leg cuffs 32 with or without elastics 33, one or more pairs of leg elastics 34, one or more elastic waistbands 36, and/or one or more acquisition materials 38. The acquisition material or materials 38 may be positioned intermediate the topsheet 26 and the absorbent core 30. An outer cover material 40, such as a nonwoven material, may cover a garment-facing side of the backsheet 28. The absorbent article 10 comprises back ears 42 in the back waist region 16. The back ears 42 may comprise fasteners 46 and may extend from the back waist region 16 of the absorbent article 10 and attach (using the fasteners 46) to the landing zone area or landing zone material 44 on a garment-facing portion of the front waist region 12 of the absorbent article 10. The absorbent article 10 also has front ears 47 in the front waist region 12. The absorbent article 10 may have a central lateral (or transverse) axis 48 and a central longitudinal axis 50. The central lateral axis 48 extends perpendicular to the central longitudinal axis 50.

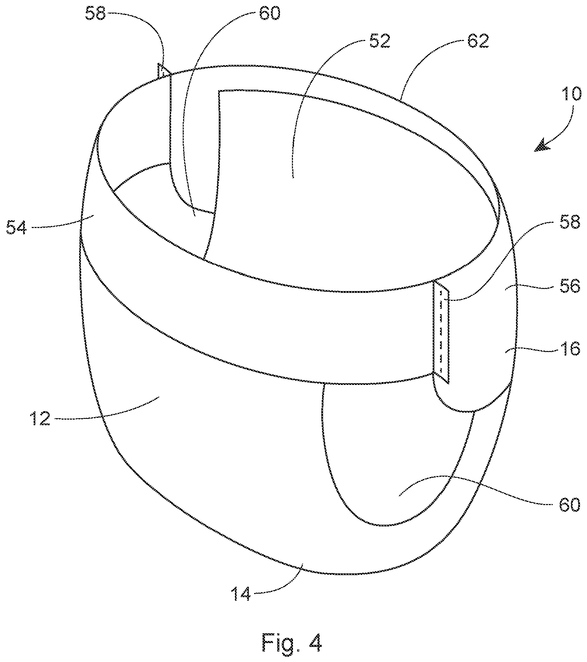

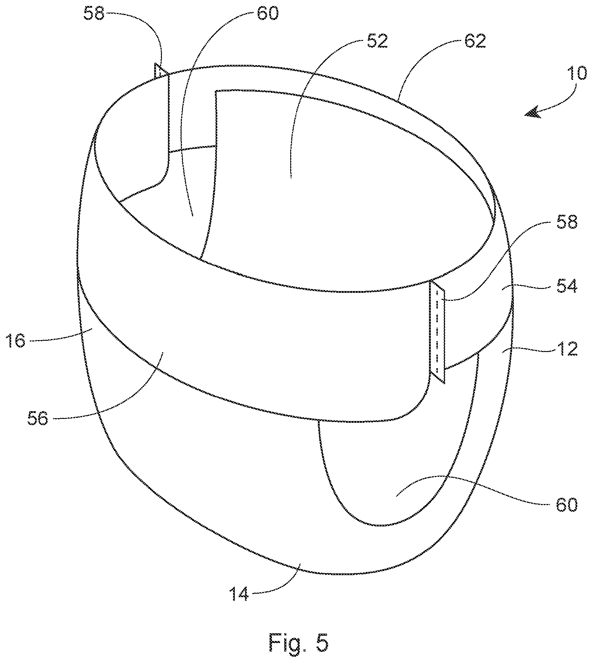

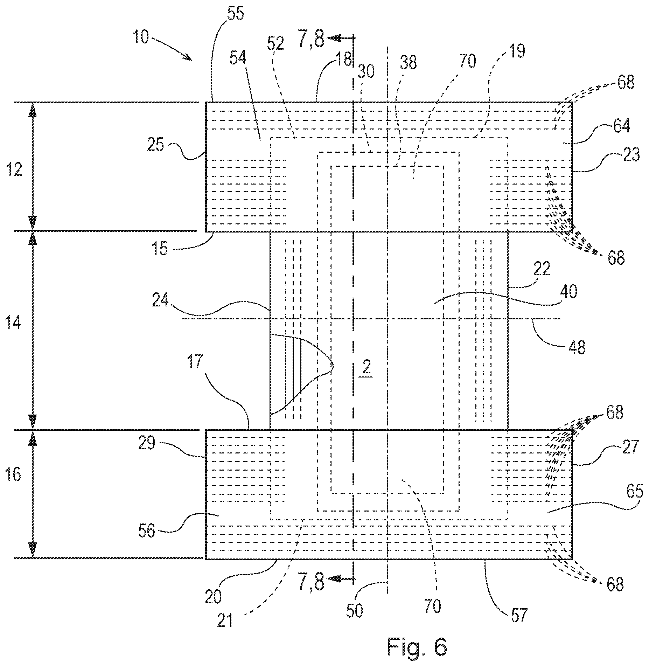

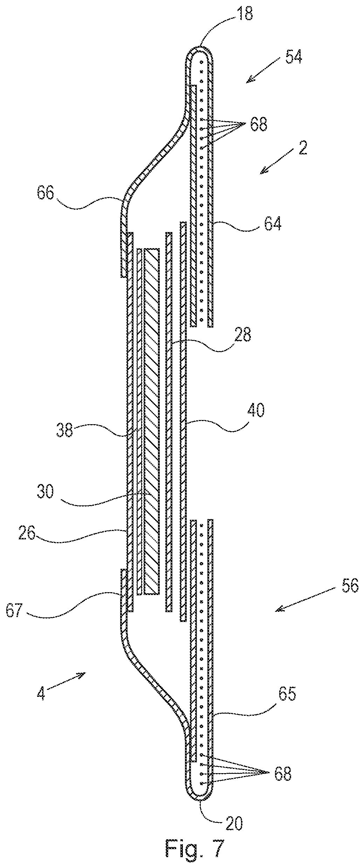

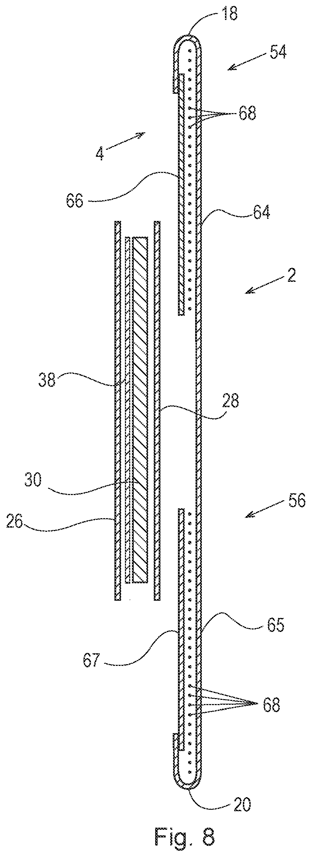

In other instances, the absorbent article may be in the form of a pant having permanent or refastenable side seams. Suitable refastenable seams are disclosed in US2014/0005020 and US9,421,137. Referring to Figs. 4-8, an example absorbent article 10 in the form of a pant is illustrated. Fig. 4 is a front perspective view of the absorbent article 10. Fig. 5 is a rear perspective view of the absorbent article 10. Fig. 6 is a plan view of the absorbent article 10, laid flat, with the garment-facing surface facing the viewer. Elements of Fig. 4-8 having the same reference number as described above with respect to Figs. 1-3 may be the same element (e.g., absorbent core 30). Fig. 7 is an example cross-sectional view of the absorbent article taken about line 7-7 of Fig. 6. Fig. 8 is an example cross-sectional view of the absorbent article taken about line 8-8 of Fig. 6. Figs. 7 and 8 illustrate example forms of front and back belts 54, 56. The absorbent article 10 may have a front waist region 12, a crotch region 14, and a back waist region 16. Each of the regions 12, 14, and 16 may be 1/3 of the length of the absorbent article 10. The absorbent article 10 may have a chassis 52 (sometimes referred to as a central chassis or central panel) comprising a topsheet 26, a backsheet 28, and an absorbent core 30 disposed at least partially intermediate the topsheet 26 and the backsheet 28, and an optional acquisition material 38, similar to that as described above with respect to Figs. 1-3. The absorbent article 10 may comprise a front belt 54 in the front waist region 12 and a back belt 56 in the back waist region 16. The chassis 52 may be joined to a wearer-facing surface 4 of the front and back belts 54, 56 or to a garment-facing surface 2 of the belts 54, 56. Side edges 23 and 25 of the front belt 54 may be joined to side edges 27 and 29, respectively, of the back belt 56 to form two side seams 58. The side seams 58 may be any suitable seams known to those of skill in the art, such as butt seams or overlap seams, for example. When the side seams 58 are permanently formed or refastenably closed, the absorbent article 10 in the form of a pant has two leg openings 60 and a waist opening circumference 62. The side seams 58 may be permanently joined using adhesives or bonds, for example, or may be refastenably closed using hook and loop fasteners, for example.

### Belts

Referring to Figs. 7 and 8, the front and back belts 54 and 56 may comprise front and back inner belt layers 66 and 67 and front and back outer belt layers 64 and 65 having an elastomeric material (e.g., strands 68 or a film (which may be apertured)) disposed at least partially therebetween. The elastic elements 68 or the film may be relaxed (including being cut) to reduce elastic strain over the absorbent core 30 or, may alternatively, run continuously across the absorbent core 30. The elastics elements 68 may have uniform or variable spacing therebetween in any portion of the belts. The elastic elements 68 may also be pre-strained the same amount or different amounts. The front and/or back belts 54 and 56 may have one or more elastic element free zones 70 where the chassis 52 overlaps the belts 54, 56. In other instances, at least some of the elastic elements 68 may extend continuously across the chassis 52.

The front and back inner belt layers 66, 67 and the front and back outer belt layers 64, 65 may be joined using adhesives, heat bonds, pressure bonds or thermoplastic bonds. Various suitable belt layer configurations can be found in US2013/0211363.

Front and back belt end edges 55 and 57 may extend longitudinally beyond the front and back chassis end edges 19 and 21 (as shown in Fig. 6) or they may be co-terminus. The front and back belt side edges 23, 25, 27, and 29 may extend laterally beyond the chassis side edges 22 and 24. The front and back belts 54 and 56 may be continuous (i.e., having at least one layer that is continuous) from belt side edge to belt side edge (e.g., the transverse distances from 23 to 25 and from 27 to 29). Alternatively, the front and back belts 54 and 56 may be discontinuous from belt side edge to belt side edge (e.g., the transverse distances from 23 to 25 and 27 to 29), such that they are discrete.

As disclosed in US7,901,393, the longitudinal length (along the central longitudinal axis 50) of the back belt 56 may be greater than the longitudinal length of the front belt 54, and this may be particularly useful for increased buttocks coverage when the back belt 56 has a greater longitudinal length versus the front belt 54 adjacent to or immediately adjacent to the side seams 58.

The front outer belt layer 64 and the back outer belt layer 65 may be separated from each other, such that the layers are discrete or, alternatively, these layers may be continuous, such that a layer runs continuously from the front belt end edge 55 to the back belt end edge 57. This may also be true for the front and back inner belt layers 66 and 67 - that is, they may also be longitudinally discrete or continuous. Further, the front and back outer belt layers 64 and 65 may be longitudinally continuous while the front and back inner belt layers 66 and 67 are longitudinally discrete, such that a gap is formed between them - a gap between the front and back inner and outer belt layers 64, 65, 66, and 67 is shown in Fig. 7 and a gap between the front and back inner belt layers 66 and 67 is shown in Fig. 8.

The front and back belts 54 and 56 may include slits, holes, and/or perforations providing increased breathability, softness, and a garment-like texture. Underwear-like appearance can be enhanced by substantially aligning the waist and leg edges at the side seams 58 (see Figs. 4 and 5).

The front and back belts 54 and 56 may comprise graphics (see e.g., 78 of Fig. 1). The graphics may extend substantially around the entire circumference of the absorbent article 10 and may be disposed across side seams 58 and/or across proximal front and back belt seams 15 and 17; or, alternatively, adjacent to the seams 58, 15, and 17 in the manner described in U.S. Pat. No. 9,498, 389 to create a more underwear-like article. The graphics may also be discontinuous.

Alternatively, instead of attaching belts 54 and 56 to the chassis 52 to form a pant, discrete side panels may be attached to side edges of the chassis 22 and 24. Suitable forms of pants comprising discrete side panels are disclosed in U.S. Pat. Nos. 6,645,190; 8,747,379; 8,372,052; 8,361,048; 6,761,711; 6,817,994; 8,007,485; 7,862,550; 6,969,377; 7,497,851; 6,849,067; 6,893,426; 6,953,452; 6,840,928; 8,579,876; 7,682,349; 7,156,833; and 7,201,744.

### Topsheet

The topsheet 26 is the part of the absorbent article 10 that is in contact with the wearer's skin. The topsheet 26 may be joined to portions of the backsheet 28, the absorbent core 30, the barrier leg cuffs 32, and/or any other layers as is known to those of ordinary skill in the art. The topsheet 26 may be compliant, soft-feeling, and non-irritating to the wearer's skin. Further, at least a portion of, or all of, the topsheet may be liquid permeable, permitting liquid bodily exudates to readily penetrate through its thickness. A suitable topsheet may be manufactured from a wide range of materials, such as porous foams, reticulated foams, apertured plastic films, woven materials, nonwoven materials, woven or nonwoven materials of natural fibers (e.g., wood or cotton fibers), synthetic fibers or filaments (e.g., polyester or polypropylene or bicomponent PE/PP fibers or mixtures thereof), or a combination of natural and synthetic fibers. The topsheet may have one or more layers. The topsheet may be apertured (Fig. 2, element 31), may have any suitable three-dimensional features, and/or may have a plurality of embossments (e.g., a bond pattern). The topsheet may be apertured by overbonding a material and then rupturing the overbonds through ring rolling, such as disclosed in US5,628,097, to Benson et al., issued on May 13, 1997 and disclosed in US2016/0136014 to Arora et al. Any portion of the topsheet may be coated with a skin care composition, an antibacterial agent, a surfactant, and/or other beneficial agents. The topsheet may be hydrophilic or hydrophobic or may have hydrophilic and/or hydrophobic portions or layers. If the topsheet is hydrophobic, typically apertures will be present so that bodily exudates may pass through the topsheet.

### Backsheet

The backsheet 28 is generally that portion of the absorbent article 10 positioned proximate to the garment-facing surface of the absorbent core 30. The backsheet 28 may be joined to portions of the topsheet 26, the outer cover material 40, the absorbent core 30, and/or any other layers of the absorbent article by any attachment methods known to those of skill in the art. The backsheet 28 prevents, or at least inhibits, the bodily exudates absorbed and contained in the absorbent core 10 from soiling articles such as bedsheets, undergarments, and/or clothing. The backsheet is typically liquid impermeable, or at least substantially liquid impermeable. The backsheet may, for example, be or comprise a thin plastic film, such as a thermoplastic film having a thickness of about 0.012 mm to about 0.051 mm. Other suitable backsheet materials may include breathable materials which permit vapors to escape from the absorbent article, while still preventing, or at least inhibiting, bodily exudates from passing through the backsheet.

### Outer Cover Material

The outer cover material (sometimes referred to as a backsheet nonwoven) 40 may comprise one or more nonwoven materials joined to the backsheet 28 and that covers the backsheet 28. The outer cover material 40 forms at least a portion of the garment-facing surface 2 of the absorbent article 10 and effectively "covers" the backsheet 28 so that film is not present on the garment-facing surface 2. The outer cover material 40 may comprise a bond pattern, apertures, and/or three-dimensional features. The outer cover material 40 may be a hydroentangled nonwoven material.

### Absorbent Core

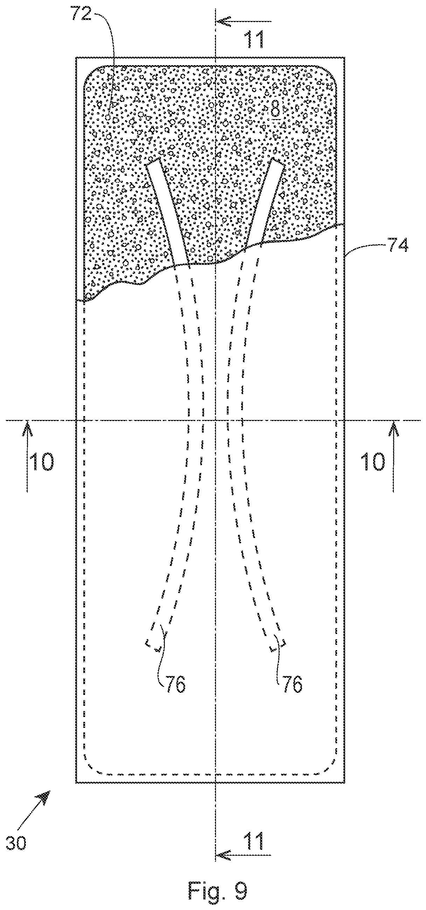

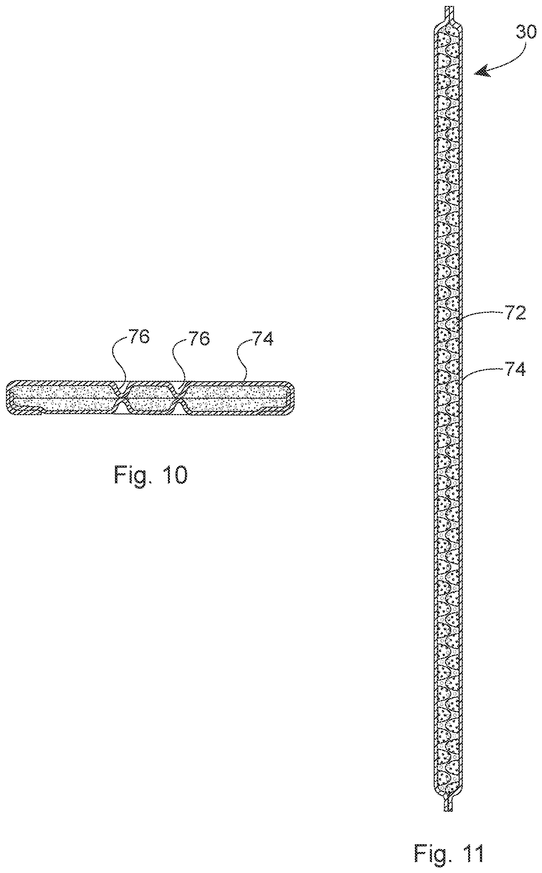

As used herein, the term "absorbent core" 30 refers to the component of the absorbent article 10 having the most absorbent capacity and that comprises an absorbent material. Referring to Figs. 9-11, in some instances, absorbent material 72 may be positioned within a core bag or a core wrap 74. The absorbent material may be profiled or not profiled, depending on the specific absorbent article. The absorbent core 30 may comprise, consist essentially of, or consist of, a core wrap, absorbent material 72, and glue enclosed within the core wrap. The absorbent material may comprise superabsorbent polymers, a mixture of superabsorbent polymers and air felt, only air felt, and/or a high internal phase emulsion foam. In some instances, the absorbent material may comprise at least 80%, at least 85%, at least 90%, at least 95%, at least 99%, or up to 100% superabsorbent polymers, by weight of the absorbent material. In such instances, the absorbent material may be free of air felt, or at least mostly free of air felt. The absorbent core periphery, which may be the periphery of the core wrap, may define any suitable shape, such as rectangular "T," "Y," "hour-glass," or "dog-bone" shaped, for example. An absorbent core periphery having a generally "dog bone" or "hour-glass" shape may taper along its width towards the crotch region 14 of the absorbent article 10.

Referring to Figs. 9-11, the absorbent core 30 may have areas having little or no absorbent material 72, where a wearer-facing surface of the core bag 74 may be joined to a garment-facing surface of the core bag 74. These areas having little or no absorbent material and may be referred to as "channels" 76. These channels can embody any suitable shapes and any suitable number of channels may be provided. In other instances, the absorbent core may be embossed to create the impression of channels. The absorbent core in Figs. 9-11 is merely an example absorbent core. Many other absorbent cores with or without channels are also within the scope of the present disclosure.

### Barrier Leg Cuffs/Leg Elastics

Referring to Figs. 1 and 2, for example, the absorbent article 10 may comprise one or more pairs of barrier leg cuffs 32 and one or more pairs of leg elastics 34. The barrier leg cuffs 32 may be positioned laterally inboard of leg elastics 34. Each barrier leg cuff 32 may be formed by a piece of material which is bonded to the absorbent article 10 so it can extend upwards from a wearer-facing surface 4 of the absorbent article 10 and provide improved containment of body exudates approximately at the junction of the torso and legs of the wearer. The barrier leg cuffs 32 are delimited by a proximal edge joined directly or indirectly to the topsheet and/or the backsheet and a free terminal edge, which is intended to contact and form a seal with the wearer's skin. The barrier leg cuffs 32 may extend at least partially between the front end edge 18 and the back end edge 20 of the absorbent article 10 on opposite sides of the central longitudinal axis 50 and may be at least present in the crotch region 14. The barrier leg cuffs 32 may each comprise one or more elastics 33 (e.g., elastic strands or strips) near or at the free terminal edge. These elastics 33 cause the barrier leg cuffs 32 to help form a seal around the legs and torso of a wearer. The leg elastics 34 extend at least partially between the front end edge 18 and the back end edge 20. The leg elastics 34 essentially cause portions of the absorbent article 10 proximate to the chassis side edges 22, 24 to help form a seal around the legs of the wearer. The leg elastics 34 may extend at least within the crotch region 14.

### Elastic Waistband

Referring to Figs. 1 and 2, the absorbent article 10 may comprise one or more elastic waistbands 36. The elastic waistbands 36 may be positioned on the garment-facing surface 2 or the wearer-facing surface 4. As an example, a first elastic waistband 36 may be present in the front waist region 12 near the front belt end edge 18 and a second elastic waistband 36 may be present in the back waist region 16 near the back end edge 20. The elastic waistbands 36 may aid in sealing the absorbent article 10 around a waist of a wearer and at least inhibiting bodily exudates from escaping the absorbent article 10 through the waist opening circumference. In some instances, an elastic waistband may fully surround the waist opening circumference of an absorbent article.

### Acquisition Materials

Referring to Figs. 1, 2, 7, and 8, one or more acquisition materials 38 may be present at least partially intermediate the topsheet 26 and the absorbent core 30. The acquisition materials 38 are typically hydrophilic materials that provide significant wicking of bodily exudates. These materials may dewater the topsheet 26 and quickly move bodily exudates into the absorbent core 30. The acquisition materials 38 may comprise one or more nonwoven materials, foams, cellulosic materials, cross-linked cellulosic materials, air laid cellulosic nonwoven materials, spunlace materials, or combinations thereof, for example. In some instances, portions of the acquisition materials 38 may extend through portions of the topsheet 26, portions of the topsheet 26 may extend through portions of the acquisition materials 38, and/or the topsheet 26 may be nested with the acquisition materials 38. Typically, an acquisition material 38 may have a width and length that are smaller than the width and length of the topsheet 26. The acquisition material may be a secondary topsheet in the feminine pad context. The acquisition material may have one or more channels as described above with reference to the absorbent core 30 (including the embossed version). The channels in the acquisition material may align or not align with channels in the absorbent core 30. In an example, a first acquisition material may comprise a nonwoven material and as second acquisition material may comprise a cross-linked cellulosic material.

### Landing Zone

Referring to Figs. 1 and 2, the absorbent article 10 may have a landing zone area 44 that is formed in a portion of the garment-facing surface 2 of the outer cover material 40. The landing zone area 44 may be in the back waist region 16 if the absorbent article 10 fastens from front to back or may be in the front waist region 12 if the absorbent article 10 fastens back to front. In some instances, the landing zone 44 may be or may comprise one or more discrete nonwoven materials that are attached to a portion of the outer cover material 40 in the front waist region 12 or the back waist region 16 depending upon whether the absorbent article fastens in the front or the back. In essence, the landing zone 44 is configured to receive the fasteners 46 and may comprise, for example, a plurality of loops configured to be engaged with, a plurality of hooks on the fasteners 46, or *vice versa.*

### Wetness Indicator/Graphics

Referring to Fig. 1, the absorbent articles 10 of the present disclosure may comprise graphics 78 and/or wetness indicators 80 that are visible from the garment-facing surface 2. The graphics 78 may be printed on the landing zone 40, the backsheet 28, and/or at other locations. The wetness indicators 80 are typically applied to the absorbent core facing side of the backsheet 28, so that they can be contacted by bodily exudates within the absorbent core 30. In some instances, the wetness indicators 80 may form portions of the graphics 78. For example, a wetness indicator may appear or disappear and create/remove a character within some graphics. In other instances, the wetness indicators 80 may coordinate (e.g., same design, same pattern, same color) or not coordinate with the graphics 78.

### Front and Back Ears

Referring to Figs. 1 and 2, as referenced above, the absorbent article 10 may have front and/or back ears 47, 42 in a taped diaper context. The back ears 42 may comprise fasteners 46 configured to engage the landing zone or landing zone area 44. The ears, or portions thereof, may be elastic or may have elastic panels. In an example, an elastic film or elastic strands may be positioned intermediate a first nonwoven material and a second nonwoven material. The elastic film may or may not be apertured. The ears may be shaped. The ears may be integral (e.g., extension of the outer cover material 40, the backsheet 28, and/or the topsheet 26) or may be discrete components attached to a chassis 52 of the absorbent article on a wearer-facing surface 4, on the garment-facing surface 2, or intermediate the two surfaces 4, 2. The front ears 47 comprise patterns as will be discussed in further detail below.

### Masking Layer

One or more masking layers or materials may be provided in the absorbent articles 10. A masking layer may be a layer that provides a cushiony feel when the absorbent article is touched from the garment-facing surface 2 or the wearer-facing surface 4. The masking layer may "mask" a grainy feel potentially caused by the absorbent material 72, such as superabsorbent polymers. The masking layer may "mask" bodily exudates from being visible when viewing the wearer-facing surface 4 or the garment-facing surface 2 of the absorbent article 10. The masking layer may have a basis weight in the range of about 15 gsm to about 50 gsm or about 15 gsm to about 40 gsm. The masking layer may comprise one or more nonwoven materials (e.g., a hydroentangled nonwoven material), foams, pulp layers, and/or other suitable materials. The masking layer may be the outer cover material 40. The masking layer may be the layer forming the garment-facing side or the wearer-facing side of the core bag 74. The masking layer may be a separate material positioned intermediate the garment-facing side of the core bag 75 and the liquid impermeable backsheet 28.

### Sensors

Referring again to Fig. 1, the absorbent articles of the present disclosure may comprise a sensor system 82 for monitoring changes within the absorbent article 10. The sensor system 82 may be discrete from or integral with the absorbent article 10. The absorbent article 10 may comprise sensors that can sense various aspects of the absorbent article 10 associated with insults of bodily exudates such as urine and/or BM (e.g., the sensor system 82 may sense variations in temperature, humidity, presence of ammonia or urea, various vapor components of the exudates (urine and feces), changes in moisture vapor transmission through the absorbent articles garment-facing layer, changes in translucence of the garment-facing layer, and/or color changes through the garment-facing layer). Additionally, the sensor system 82 may sense components of urine, such as ammonia or urea and/or byproducts resulting from reactions of these components with the absorbent article 10. The sensor system 82 may sense byproducts that are produced when urine mixes with other components of the absorbent article 10 (e.g., adhesives, agm). The components or byproducts being sensed may be present as vapors that may pass through the garment-facing layer. It may also be desirable to place reactants in the absorbent article that change state (e.g. color, temperature) or create a measurable byproduct when mixed with urine or BM. The sensor system 82 may also sense changes in pH, pressure, odor, the presence of gas, blood, a chemical marker or a biological marker or combinations thereof. The sensor system 82 may have a component on or proximate to the absorbent article that transmits a signal to a receiver more distal from the absorbent article, such as an iPhone, for example. The receiver may output a result to communicate to the caregiver a condition of the absorbent article 10. In other instances, a receiver may not be provided, but instead the condition of the absorbent article 10 may be visually or audibly apparent from the sensor on the absorbent article.

### Packages

The absorbent articles of the present disclosure may be placed into packages. The packages may comprise polymeric films and/or other materials. Graphics and/or indicia relating to properties of the absorbent articles may be formed on, printed on, positioned on, and/or placed on outer portions of the packages. Each package may comprise a plurality of absorbent articles. The absorbent articles may be packed under compression so as to reduce the size of the packages, while still providing an adequate amount of absorbent articles per package. By packaging the absorbent articles under compression, caregivers can easily handle and store the packages, while also providing distribution savings to manufacturers owing to the size of the packages.

### Arrays

"Array" means a display of packages comprising disposable absorbent articles of different article constructions (e.g., different elastomeric materials [compositionally and/or structurally] in the side panels, side flaps and/or belts flaps, different graphic elements, different product structures, fasteners or lack thereof). The packages may have the same brand and/or sub-brand and/or the same trademark registration and/or having been manufactured by or for a common manufacturer and the packages may be available at a common point of sale (e.g. oriented in proximity to each other in a given area of a retail store). An array is marketed as a line-up of products normally having like packaging elements (e.g., packaging material type, film, paper, dominant color, design theme, etc.) that convey to consumers that the different individual packages are part of a larger line-up. Arrays often have the same brand, for example, "Huggies," and same sub-brand, for example, "Pull-Ups." A different product in the array may have the same brand "Huggies" and the sub-brand "Little Movers." The differences between the "Pull-Ups" product of the array and the "Little Movers" product in the array may include product form, application style, different fastening designs or other structural elements intended to address the differences in physiological or psychological development. Furthermore, the packaging is distinctly different in that "Pull-Ups" is packaged in a predominately blue or pink film bag and "Little Movers" is packaged in a predominately red film bag.

Further regarding "Arrays," as another example an array may be formed by different products having different product forms manufactured by the same manufacturer, for example, "Kimberly-Clark", and bearing a common trademark registration for example, one product may have the brand name "Huggies," and sub-brand, for example, "Pull-Ups." A different product in the array may have a brand/sub-brand "Good Nites" and both are registered trademarks of The Kimberly-Clark Corporation and/or are manufactured by Kimberly-Clark. Arrays also often have the same trademarks, including trademarks of the brand, sub-brand, and/or features and/or benefits across the line-up. "On-line Array" means an "Array" distributed by a common on-line source.

### Sanitary Napkin

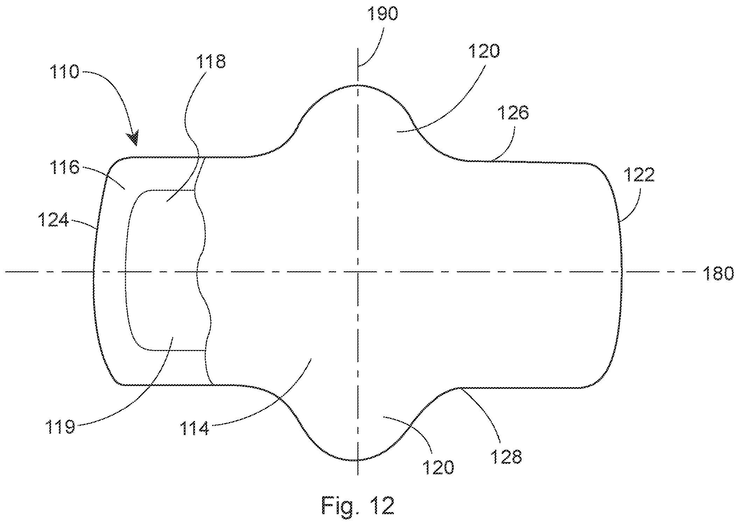

Referring to Fig. 12, an absorbent article of the present disclosure may be a sanitary napkin 110. The sanitary napkin 110 may comprise a liquid permeable topsheet 114, a liquid impermeable, or substantially liquid impermeable, backsheet 116, and an absorbent core 118. The liquid impermeable backsheet 116 may or may not be vapor permeable. The absorbent core 118 may have any or all of the features described herein with respect to the absorbent core 30 and, in some forms, may have a secondary topsheet 119 (STS) instead of the acquisition materials disclosed above. The STS 119 may comprise one or more channels, as described above (including the embossed version). In some forms, channels in the STS 119 may be aligned with channels in the absorbent core 118. The sanitary napkin 110 may also comprise wings 120 extending outwardly with respect to a longitudinal axis 180 of the sanitary napkin 110. The sanitary napkin 110 may also comprise a lateral axis 190. The wings 120 may be joined to the topsheet 114, the backsheet 116, and/or the absorbent core 118. The sanitary napkin 110 may also comprise a front edge 122, a back edge 124 longitudinally opposing the front edge 122, a first side edge 126, and a second side edge 128 longitudinally opposing the first side edge 126. The longitudinal axis 180 may extend from a midpoint of the front edge 122 to a midpoint of the back edge 124. The lateral axis 190 may extend from a midpoint of the first side edge 128 to a midpoint of the second side edge 128. The sanitary napkin 110 may also be provided with additional features commonly found in sanitary napkins as is known in the art.

### Examples Cross-sections of Absorbent Articles

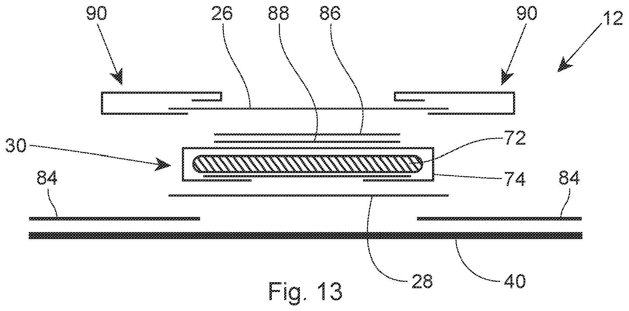

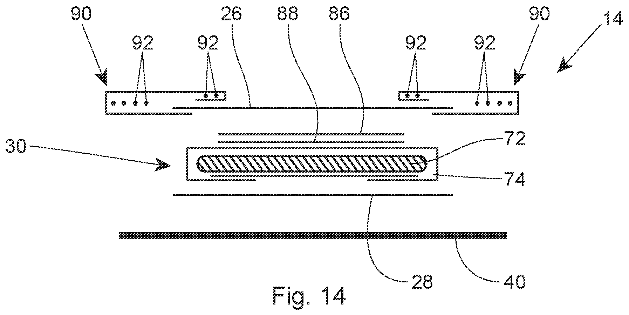

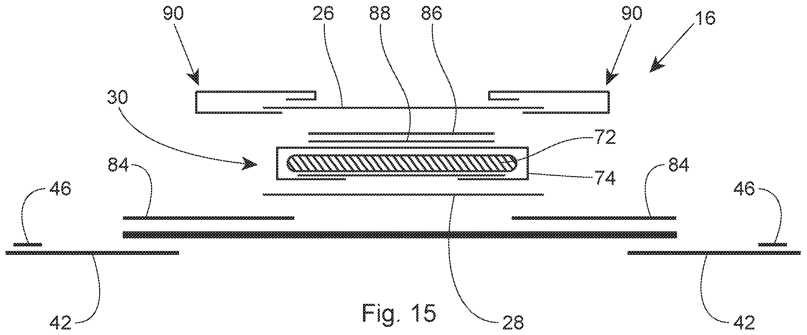

Figs. 13-15 illustrate example cross-sectional views of absorbent articles within the scope of the present disclosure. Fig. 13 is an example cross-sectional view taken within a front waist region 12 of an absorbent article. Fig. 14 is an example cross-sectional view taken within a crotch region 14 of an absorbent article. Fig. 15 is an example cross-sectional view taken within a back waist region 16 of an absorbent article. In Figs. 13-15, an outer cover material is element 40, a liquid permeable topsheet is element 26, opacity patches are elements 84, a liquid impermeable backsheet is element 28, an absorbent core is element 30, with the core bag being element 74, an absorbent material is element 72, and a distribution material is element 86. The distribution material 86 may comprise cross-linked cellulosic material and may be optional. An acquisition material is element 88. A liquid permeable topsheet is element 26. Barrier leg cuffs are elements 90. Elastics in the barrier leg cuffs are elements 92. Back ears are elements 42. Fasteners on the back ears 42 are elements 46. Construction glues and/or bonds between the various layers and/or components have been removed for clarity. Other cross-sectional configurations known to those of skill in the art are also within the scope of the present disclosure.

### Bio-Based Content for Components

Components of the absorbent articles described herein may at least partially be comprised of bio-based content as described in U.S. Pat. Appl. No. 2007/0219521A1. For example, the superabsorbent polymer component may be bio-based via their derivation from bio-based acrylic acid. Bio-based acrylic acid and methods of production are further described in US2007/0219521 and US8,703,450; US9,630,901 and US9,822,197. Other components, for example nonwoven and film components, may comprise bio-based polyolefin materials. Bio-based polyolefins are further discussed in US2011/0139657, US2011/0139658, US2011/0152812, and US2016/0206774, and US 9,169,366. Example bio-based polyolefins for use in the present disclosure comprise polymers available under the designations SHA7260^{™}, SHE150^{™}, or SGM9450F^{™} (all available from Braskem S.A.).

An absorbent article component may comprise a bio-based content value from about 10% to about 100%, from about 25% to about 100%, from about 40% to about 100%, from about 50% to about 100%, from about 75% to about 100%, or from about 90% to about 100%, for example, using ASTM D6866-10, method B.

### Recycle Friendly and Bio-Based Absorbent Articles

Components of the absorbent articles described herein may be recycled for other uses, whether they are formed, at least in part, from recyclable materials. Examples of absorbent article materials that may be recycled are nonwovens, films, fluff pulp, and superabsorbent polymers. The recycling process may use an autoclave for sterilizing the absorbent articles, after which the absorbent articles may be shredded and separated into different byproduct streams. Example byproduct streams may comprise plastic, superabsorbent polymer, and cellulose fiber, such as pulp. These byproduct streams may be used in the production of fertilizers, plastic articles of manufacture, paper products, viscose, construction materials, absorbent pads for pets or on hospital beds, and/or for other uses. Further details regarding absorbent articles that aid in recycling, designs of recycle friendly diapers, and designs of recycle friendly and bio-based component diapers, are disclosed in US2019/0192723, published on June 27, 2019.

### Front Ears With Patterns

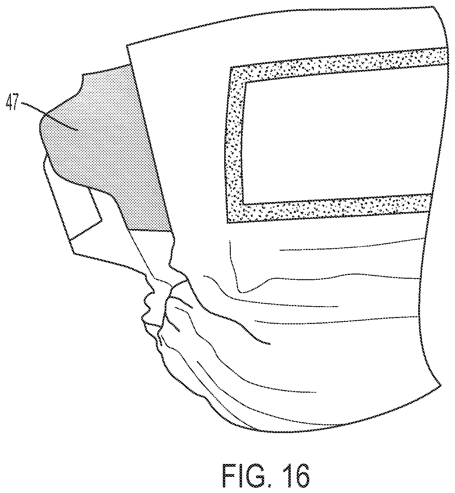

As referenced above, the absorbent articles of the present disclosure, especially taped diapers, comprise front ears 47 (see Figs. 1 and 2). The front ears 47 may comprise one or more patterns. The patterns comprise three-dimensional features. The patterns may also comprise apertures and may be free of printing and/or ink. The front ears 47 may comprise patterns that coordinate or do not coordinate with patterns of other materials or nonwoven materials on other components of the absorbent articles, such as back ears, outer cover nonwoven materials, topsheets, and/or landing zones, for example. The patterns may also coordinate or not coordinate with graphics or printing on the absorbent articles. The patterns of the front ears 47 may also comprise visually distinguishable zones, two of more regions of different textures, two or more regions of different opacities, two or more regions of different colors, two or more regions of color intensities, two or more regions of different sheens, apertures, and/or apertures, for example. The three-dimensional features of the pattern cover only a portion of the front ears that forms a grasp tab. Figures. 16-32 illustrate some examples of the front ears 47 with patterns disclosed herein.

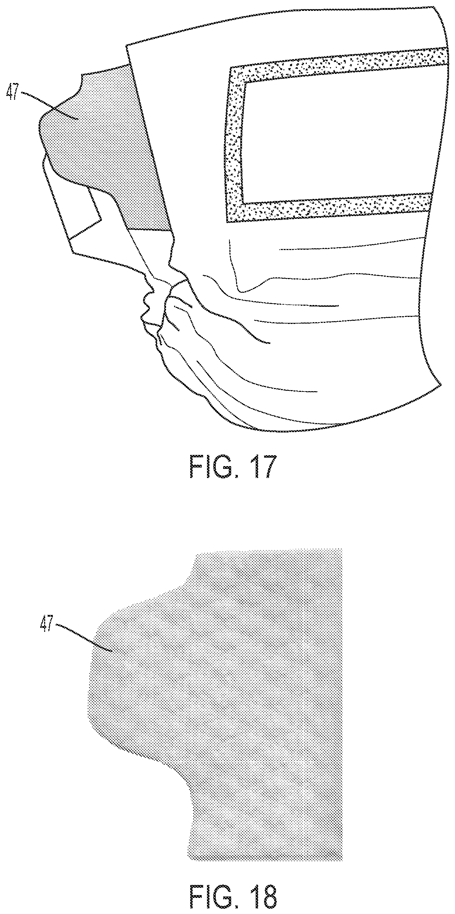

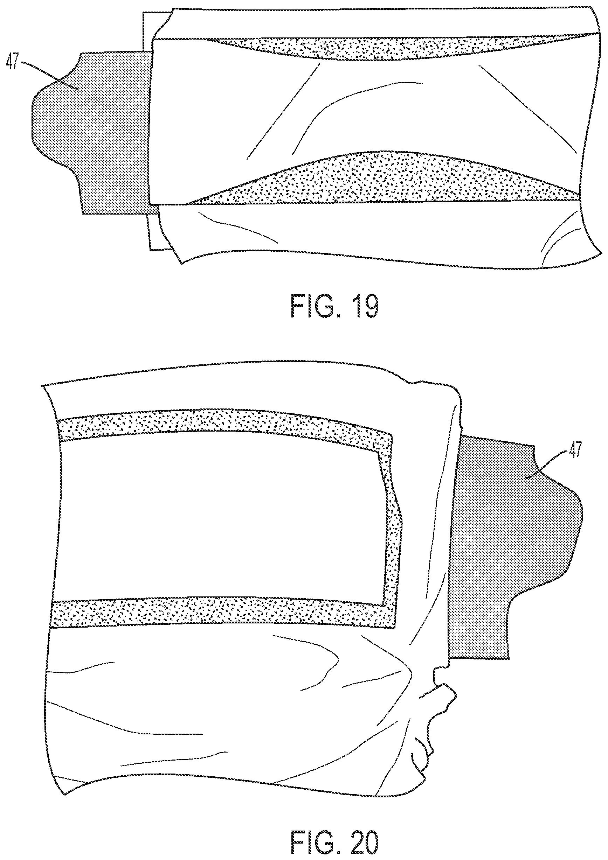

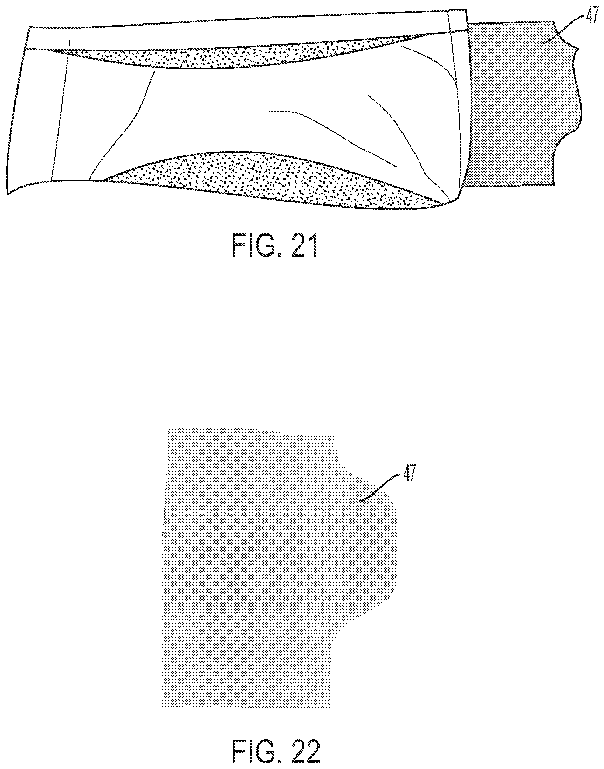

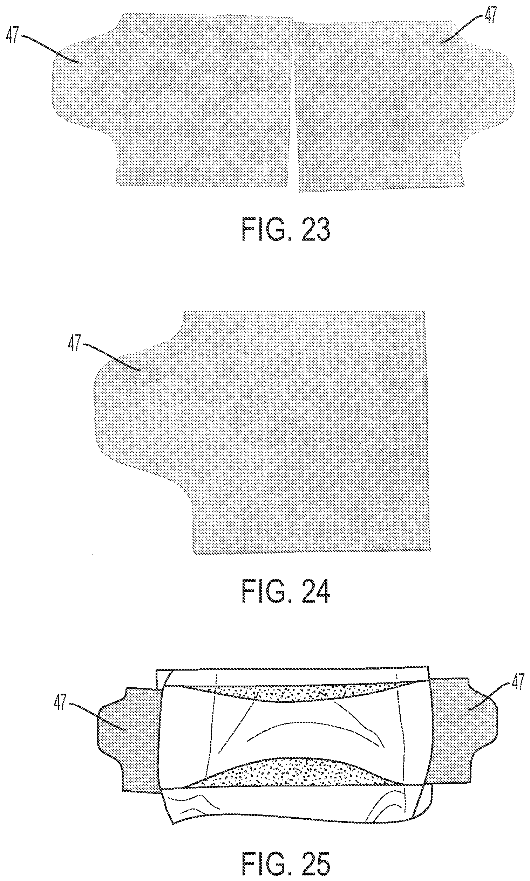

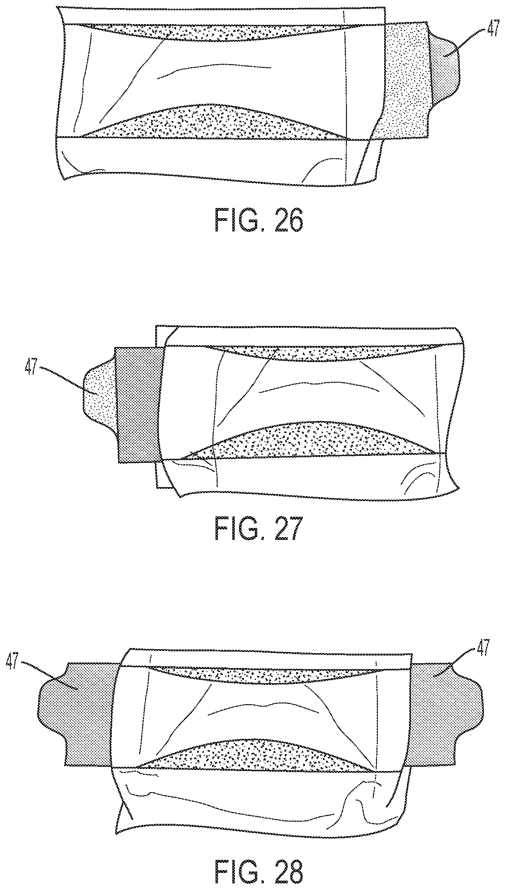

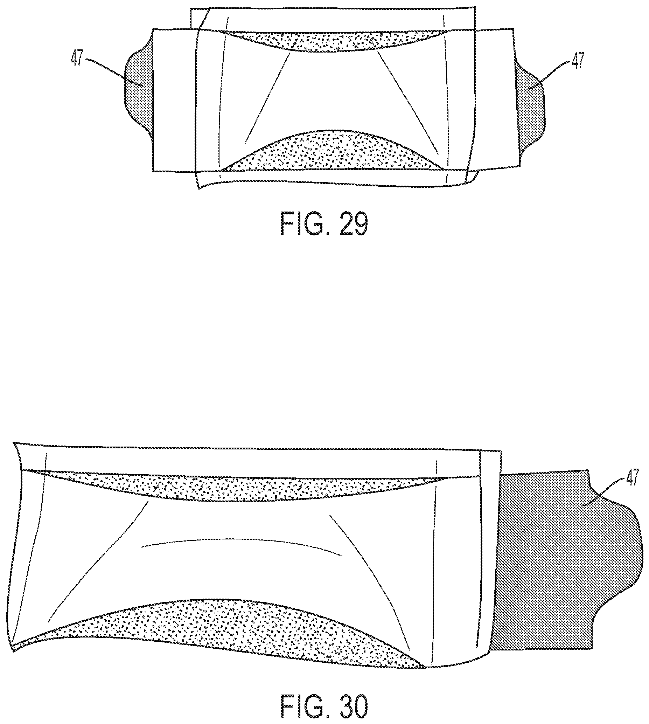

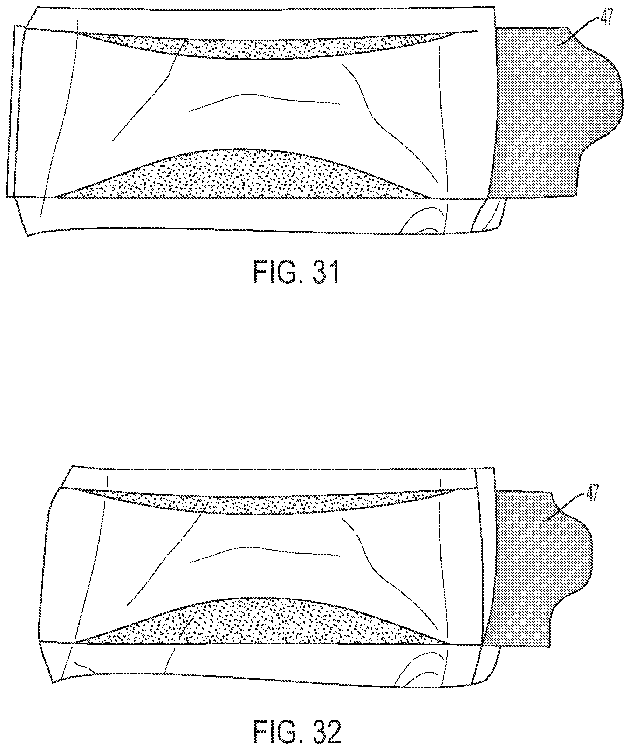

Fig. 16 is a perspective view of a portion of an absorbent article comprising a front ear 47 with a pattern comprising three-dimensional features. Fig. 17 is a perspective view of a portion of an absorbent article comprising a front ear 47 with a pattern comprising three-dimensional features. Fig. 18 is a plan view of a front ear 47 with a pattern comprising three-dimensional features. As can be seen in Fig. 18, the three-dimensional features may have regions having different heights and/or sizes. The regions with lower heights may be continuous and the regions with higher heights may be discrete, or *vice versa.* Fig. 19 is a plan view of a portion of an absorbent article comprising a front ear 47 with a pattern comprising three-dimensional features. As can be seen in Fig. 19, the three-dimensional features may have regions having different heights and/or sizes. The regions with lower heights may be continuous and the regions with higher heights may be discrete, or *vice versa.* Any of the front ear patterns disclosed herein may also comprise apertures, embossments, other visually distinguishable pattern(s), and/or point bonds to provide a signal of breathability or breathability (for apertures). Fig. 20 is a perspective view of a portion of an absorbent article comprising a front ear 47 with a pattern comprising visually distinguishable zones. The visually distinguishable zones may have regions of different sizes, shapes, opacities, sheens, and/or colors, for example. Fig. 21 is a plan view of a portion of an absorbent article comprising a front ear 47 with a pattern comprising three-dimensional features. The three-dimensional features may have raised regions and non-raised regions that both have a wavy line pattern. Fig. 22 is a plan view of a front ear 47 with a pattern comprising visually distinguishable zones. Fig. 23 is a plan view of front ears 47 with patterns comprising visually distinguishable zones. Fig. 24 is a plan view of a front ear 47 with a pattern comprising visually distinguishable zones. Fig. 25 is a plan view of a portion of an absorbent article comprising front ears 47 with patterns comprising three-dimensional features. Fig. 26 is a plan view of a portion of an absorbent article comprising a front ear 47 with a pattern comprising three-dimensional features covering only a portion of the front ear. The portion that is covered by the three-dimensional features may be a grasp tab region. Fig. 27 is a plan view of a portion of an absorbent article comprising a front ear 47 with a pattern comprising three-dimensional features covering only a portion of the front ear. Fig. 28 is a plan view of a portion of an absorbent article comprising front ears 47 with patterns comprising three-dimensional features and with the front ears having a non-white color or tint. Fig. 29 is a plan view of a portion of an absorbent article comprising front ears 47 with patterns comprising three-dimensional features and with the front ears having a non-white color or tint, wherein only a portion of the front ears have the three-dimensional features. Fig. 30 is a plan view of a portion of an absorbent article comprising a front ear 47 with a pattern comprising visually distinguishable zones with apertured areas and non-apertured areas. Fig. 31 is a plan view of a portion of an absorbent article comprising a front ear 47 with a pattern comprising visually distinguishable zones with apertured areas and non-apertured areas. Fig. 32 is a plan view of a portion of an absorbent article comprising a front ear 47 with a pattern comprising visually distinguishable zones with apertured areas and non-apertured areas.

The absorbent articles of the invention comprise a front waist region, a back waist region, a liquid permeable topsheet, a liquid impermeable backsheet, and an absorbent core positioned at least partially intermediate the topsheet and the backsheet. The absorbent core comprises an absorbent material. The absorbent material may be as described above. The absorbent article comprises a pair of back ears in the back waist region and a pair of front ears in the front waist region. The front ears comprise a patterns that comprises three-dimensional features, the three-dimensional features comprising two or more projections and a continuous planar area surrounding the two or more projections. The three-dimensional features are only present on a portion of the front ears, such as in a grasp region or tab for ease in gripping or in signaling the grasp region to a caregiver. The pattern comprises three-dimensional features and the three-dimensional features form the visually distinguishable zones. Any of the front ears described herein may comprise apertures or may be free of apertures.

The pattern may comprise the visually distinguishable zones. The visually distinguishable zones may comprise apertured areas and nonapertured areas. The visually distinguishable zones may comprise first regions having a first opacity and second regions having a second, different opacity. The visually distinguishable zones may comprise first regions having a first height and second regions having a second, different height. The visually distinguishable zones may comprise first regions having a first size and second regions having a second, different size. The visually distinguishable zones may comprise first regions having a first basis weight and second regions having a second, different basis weight, with both the first and second basis weights being greater than zero. The visually distinguishable zones may comprise first regions having a first volumetric density and second regions having a second, different volumetric density, with both the first and second volumetric densities both being greater than zero. The visually distinguishable zones may comprise first regions having a first caliper and second regions having a second, different caliper, with the first and second calipers both being greater than zero. The visually distinguishable zones may comprise first regions having a first color and second regions having a second, different color. The visually distinguishable zones may comprise first regions having a first color intensity and second regions having a second, different intensity. The visually distinguishable zones may comprise first regions having a first sheen and second regions having a second different sheen. Any of the patterns described herein may be free of printing or ink.

The front ears may be more stretchy or have a lower modulus in one direction compared to another perpendicular direction. A first direction may be parallel to the central lateral axis and a second direction may be parallel to the central longitudinal axis.

### Opacity Test Method

Opacity by contrast ratio measurements are made using a 0°/45° spectrophotometer with adjustable apertures capable of making standard CIE color measurements using XYZ coordinates. An example of a suitable spectrophotometer is the Labscan XE (available from Hunter Associates Laboratory, Inc., Reston, VA, or equivalent). Measurements are conducted on a single layer of nonwoven test material. All testing is performed in a room maintained at a temperature of 23° C ± 2.0° C and a relative humidity of 50% ± 2% and samples are conditioned under the same environmental conditions for at least 2 hours prior to testing.

Obtain a sample nonwoven substrate. To obtain a sample from an absorbent article, first identify the portion of the absorbent article of interest. Carefully remove the sample of nonwoven material from the absorbent article. A cryogenic spray (such as Cyto-Freeze, Control Company, Houston TX) can be used to remove the sample from the underlying and overlaying layers if necessary. Identify a first region(s) and a second region(s) of the nonwoven substrate. The first region(s) and the second region(s) are visually different from each other in at least one of color, opacity, tone, shade and/or reflectance. If the sample at the testing site contains any holes, tears, or other physical deformations, another site is to be selected. Ensure that all adhesive have been completely removed from the testing site. Obtain a sufficient quantity of the nonwoven substrate sample material such that ten replicate measurements can be made on each first region(s) and second region(s).

To measure Opacity, select the disk with the largest measurement port size that can fit within the selected first region. Calibrate and standardize the instrument per the vendor instructions using the standard white and black tiles provided by the vendor. Set the spectrophotometer to use the CIE XYZ color space with a D65 standard illumination, a 10° observer, and set the UV filter to nominal. Place the garment-facing surface of the test sample over the aperture and ensure that the entire aperture opening is covered by the testing site graphic. Place the standard white tile directly against the back side of the sample, take a reading and record the Y value as Y_{white backing} to the nearest 0.01 units. Without moving the position of the test sample, remove the standard white tile and replace it with the black standard tile. Take a reading and record the Y value as Y_{black backing} to the nearest 0.01 units. Calculate Opacity by dividing the Y_{black backing} value by the Y_{whit backing} value and then multiply by 100. Record Opacity to the nearest 0.1 percent.

In like fashion, repeat the testing procedure for a total of ten on different locations within the first region(s). Calculate the arithmetic mean for Opacity obtained from all ten measurements and report to the nearest 0.1 percent as First Opacity.

Using the same prepared patterned nonwoven substrate samples, repeat the testing procedure on ten replicate second region(s). Calculate the arithmetic mean for Opacity obtained from all ten measurements and report to the nearest 0.1 percent as Second Opacity.

Report the difference between the First Opacity and the Second Opacity as Δ Opacity to the nearest 0.1 percent.

## Claims

1. An absorbent article (10) comprising:
a front waist region (12);
a back waist region (16);
a liquid permeable topsheet (26);
a liquid impermeable backsheet (28);
an absorbent core (30) positioned at least partially intermediate the topsheet and the backsheet, the absorbent core comprising an absorbent material;
a pair of back ears (42) in the back waist region; and
a pair of front ears (47) in the front waist region;
wherein the front ears comprise a pattern, **characterized in that** the pattern comprises three-dimensional features only present on a portion of the front ears, wherein the three-dimensional features comprise two or more projections and a continuous planar area surrounding the two or more projections, and wherein the portion forms a grasp tab on the front ears.

2. The absorbent article of claim 1, wherein each of the front ears comprise apertures.

3. The absorbent article of any one of the preceding claims, wherein the pattern is free of printing, wherein the absorbent article is a taped diaper, and wherein the back ears comprise ultrasonic bonds.

## Patentansprüche

1. Absorptionsartikel (10), umfassend:
einen vorderseitigen Taillenbereich (12);
einen rückseitigen Taillenbereich (16);
eine flüssigkeitsdurchlässige Oberschicht (26);
eine flüssigkeitsundurchlässige Unterschicht (28);
einen Absorptionskern (30), der wenigstens teilweise zwischen der Oberschicht und der Unterschicht angeordnet ist, der Absorptionskern umfassend ein Absorptionsmaterial;
ein Paar von rückseitigen Seitenlappen (42) in dem rückseitigen Taillenbereich; und
ein Paar von vorderseitigen Seitenlappen (47) in dem vorderseitigen Taillenbereich;
wobei die vorderseitigen Seitenlappen ein Muster umfassen,
**dadurch gekennzeichnet, dass** das Muster dreidimensionale Merkmale umfasst, die nur auf einem Abschnitt der vorderseitigen Seitenlappen vorhanden sind, wobei die dreidimensionalen Merkmale zwei oder mehr Vorsprünge und einen kontinuierlichen ebenen Bereich umfassen, der die zwei oder mehr Vorsprünge umgibt, und wobei der Abschnitt eine Grifflasche an den vorderseitigen Seitenlappen ausbildet.

2. Absorptionsartikel nach Anspruch 1, wobei jeder der vorderseitigen Seitenlappen Öffnungen umfasst.

3. Absorptionsartikel nach einem der vorstehenden Ansprüche, wobei das Muster keinen Aufdruck besitzt, wobei der Absorptionsartikel eine Windel mit Klebeband ist und wobei die rückseitigen Seitenlappen Ultraschallbindungen umfassen.

## Revendications

1. Article absorbant (10) comprenant :
une région de taille avant (12) ;
une région de taille arrière (16) ;
une feuille de dessus perméable aux liquides (26) ;
une feuille de fond imperméable aux liquides (28) ;
une âme absorbante (30) positionnée au moins partiellement entre la feuille de dessus et la feuille de fond, l'âme absorbante comprenant un matériau absorbant ;
une paire de pattes arrière (42) dans la région de taille arrière ; et
une paire de pattes avant (47) dans la région de taille avant ;
dans lequel les pattes avant comprennent un motif, **caractérisé en ce que** le motif comprend des caractéristiques tridimensionnelles présentes uniquement sur une partie des pattes avant, dans lequel les caractéristiques tridimensionnelles comprennent deux saillies ou plus et une zone plane continue entourant les deux saillies ou plus, et dans lequel la partie forme une languette de préhension sur les pattes avant.

2. Article absorbant selon la revendication 1, dans lequel chacune des pattes avant comprend des ouvertures.

3. Article absorbant selon l'une quelconque des revendications précédentes, dans lequel le motif est exempt d'impression, dans lequel l'article absorbant est une couche à rubans, et dans lequel les pattes arrière comprennent des liaisons par ultrasons.
